# EUROPEAN PATENT SPECIFICATION

(11) **EP 0 483 714 B1**
(45) Date of publication and mention of the grant of the patent: **03.05.1995**
(21) Application number: 91118328.3
(22) Date of filing: 28.10.1991
(51) Int. Cl.: C07C 229/22, C07C 227/02

(54) **A method of preparing an acid additional salt of delta-aminolevulinic acid**
Ein Verfahren zur Herstellung von einem säureergänzenden Salz von delta-Aminolevulinsäure
Un procédé pour la préparation d'un sel d'acide additionnel de l'acide delta-aminolévulique

(30) Priority: 29.10.1990 JP 288481/90; 15.02.1991 JP 22372/91
(43) Date of publication of application: 06.05.1992
(73) Proprietor: Japan Tobacco Inc., Shinagawa-ku, Tokyo 140 (JP)
(72) Inventor: Ebata, Takashi, c/o JAPAN TOBACCO INC., Midori-ku, Yokohama-shi (JP); Kawakami, Hiroshi, c/o JAPAN TOBACCO INC., Midori-ku, Yokohama-shi (JP); Matsumoto, Katsuya, c/o JAPAN TOBACCO INC., Midori-ku, Yokohama-shi (JP); Koseki, Koshi, c/o JAPAN TOBACCO INC., Midori-ku, Yokohama-shi (JP); Matsushita, Hajime, c/o JAPAN TOBACCO INC., Midori-ku, Yokohama-shi (JP)
(74) Representative: Reinhard - Skuhra - Weise & Partner

(56) References cited:
- GB-A- 1 358 477
- US-A- 4 325 877
- TETRAHEDRON LETTERS, vol. 25, no. 28, 1984, OXFORD GB pages 2977 - 2980; A PFALTZ ET.AL.: 'Synthesis of alpha-Aminoketones via Selective Reduction of Acyl Cyanides'
- JOURNAL OF THE CHEMICAL SOCIETY,CHEMICAL, COMMUNICATIONS vol. 1978, no. 17, 6 September 1978, CAMBRIDGE,GB pages 753-4; J.KARBAN ET.AL.: 'A Simple Route to alpha-Aminoketones and Related Derivatives by Dianion Acylation Reactions;an Improved Preparation of delta-Aminolevulinic Acid'
- AGRICULTURAL AND BIOLOGICAL CHEMISTRY, vol. 55, no. 6, July 1991, TOKYO,JP pages 1687 - 8; H.KAWAKAMI ET.AL.:'A New Synthesis of 5-Aminolevulinic Acid'

## Description

The present invention relates to a method of preparing an acid addition salt of δ-aminolevulinic acid. The acid additional salt of δ-aminolevulinic acid is readily converted by neutralization to δ-aminolevulinic acid. δ-aminolevulinic acid is very unstable chemically so that it has been utilized in a form of an acid addition salt generally for storage and transportation.

δ-aminolevulinic acid has been known as a precursor of Vitamin B₁₂, heme and chlorophyll. Also it has been reported by C.A. Rebaiz et al. of Illinois University, U.S.A. to have a selective herbicidal effect (Enzyme Microb. Technol., Vol. 6, P 390 (1984)).

As methods of preparing an acid addition salt of δ-aminolevulinic acid, which is a synthetic intermediate of δ-aminolevulinic acid, there have been known several methods. For example, L. Pichat et al. have proposed a method of converting δ-bromolevulinate into a δ-phthalimide derivative, and deriving further the derivative to an acid additional salt of δ-aminolevulinic acid (Bull. Soc. Chim. Fr., 1750 (1956)).

S.I. Beale et al. have reported a method of synthesizing an acid addition salt of δ-aminolevulinic acid through a non-enzymic transamination of 4,5-δ-dioxolevulinic acid which is prepared by using δ-bromolevulinic acid as a start material (Phytochemistry, Vol. 18, 441 (1979)).

Further another method has been proposed by A. Pfaltz, wherein an acid addition salt of δ-aminolevulinic acid is synthesized by reducing a ketonitrile compound in the presence of zinc and acetic acid (Tetrahedoron Lett., Vol. 25, No. 28, 2977 (1984)).

Finally, GB-A-1358477 discloses also a process for the preparation of addition salt of δ-aminolevulinic acid, which process does not use the tetrahydrofurfurylamine as starting material.

These conventional methods of preparing an acid addition salt of δ-aminolevulinic acid, however, have been suffering from the following drawbacks: none of the above mentioned methods can produce δ-aminolevulinic acid at a high efficiency industrially; the production cost of the acid addition salt is rather high because of the expensive raw material or complicated processes; and further pollusive industrial wastes are yielded.

The object of the present invention is to provide a method of preparing an acid addition salt of δ-aminolevulinic acid, which salt can be prepared from a cheap and easily available raw material in a high yield.

Another object of the present invention is to provide a method of preparing an acid addition salt of δ-aminolevulinic acid in simple processes nearly free from the pollusive wastes and in an industrially practicable way.

The present inventors have researched and studied to achieve the objects of the present invention and have found that an acid addition salt of δ-aminolevulinic acid can be prepared by utilizing cheap and readily available tetrahydrofurfurylamine as a starting material through simple processes.

Namely the present inventive is, as shown by the following scheme (I) method of preparing an acid additional salt (I) of δ-aminolevulinic acid comprising:
(a) introducing an amino-protecting group into an amino group of tetrahydrofurfurylamine (IV), thereby obtaining a compound (III);
(b) oxidizing carbon atoms locating at first- and fourth-positions of the obtained compound (III), thereby obtaining a compound (II); and
(c) deprotecting the amino-protecting group of the obtained compound (II) by an acid, thereby obtaining the compound (I).

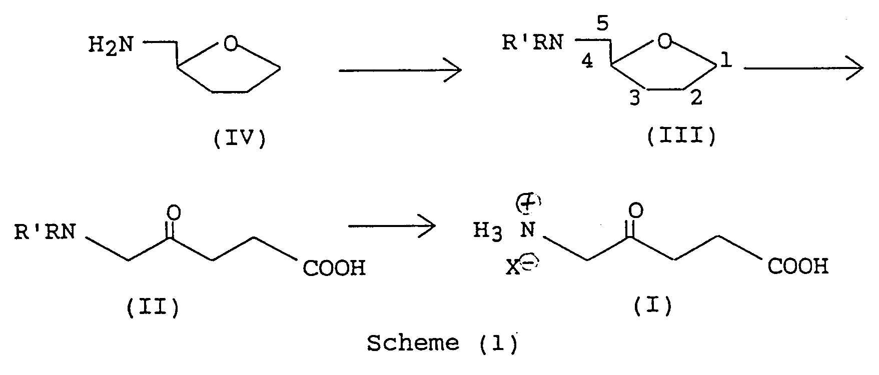

In the scheme (I), R and R' respectively represent a common amino-protecting group or a hydrogen atom and at least one of R and R' is a common amino-protecting group. When both of R and R' are the said amino-protecting group, they may be linked together to form a ring. X represents a monovalent organic or inorganic acid radical.

Herebelow detail description will be made on the method of preparing an acid addition salt of δ-aminolevulinic acid of the present invention.

In the present invention, cheap and readily available tetrahydrofurfurylamine (IV) will be used as a starting material.

The amino-protecting group in the step (a) may not be particularly restricted so far as it can be commonly employed as an amino-protecting group. For example, acyl groups and siliyl groups may be applicable as the amino-protecting groups. Regarding R and R', both of them may be an amino-protecting groups as described above or either one may be said amino-protecting group while the other is hydrogen atom. Also R and R' may be an amino-protecting group in which they are connected with each other to form a ring, such as phthalimide.

The oxidation of carbon atoms of first- and fourth-positions in the step (b) can be performed by the oxidizing reaction using ruthenium oxide as a catalyst or the oxidizing reaction using chromic acid series oxidizing agents.

In the oxidation using ruthenium oxide or ruthenium trichloride as a catalyst described above, said ruthenium may be e.g., ruthenium tetraoxide and ruthenium dioxide. The oxidation may be conducted by using such ruthenium oxide or ruthenium trichloride with together a strong oxidizing agent, such as, for instance, sodium periodate, potassium periodate, sodium hypochlorite, sodium bromate, etc., in an appropriate solvent at room temperature with stirring overnight. The above mentioned appropriate solvent may include e.g., a mixture solvent consisting of carbon tetrachloride, acetonitrile and water, but the present invention may not be limited by this particularly.

While oxidation by chromic acid series oxidizing agents may be performed by using chromic acid series oxidizing agent such as chromium trioxide and t-butyl chromate, etc, in an appropriate solvent, for instance, an organic solvent such as acetone.

The above explained oxidation reactions will proceed via a reaction intermediate (V) as expressed by the following formula. Said reaction intermediate (V) may possibly be isolated during oxidation reaction. The similar oxidation as in the step (b) can be conducted to yield the compound (II) from the reaction intermediate (V). 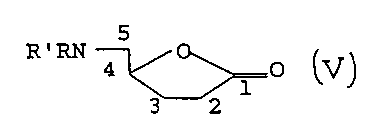
Deprotection reaction in which the amino-protecting group is separated from the compound (II) in the step (c), will be carried out in a suitable solvent by using an acid. Acids to be used for the deprotection reaction may be organic or inorganic acid, which is to react with the amino group of δ-aminolevulinic acid to yield an acid addition salt. Examples of such organic acids may e.g., include acetic acid, trifluoroacetic acid and paratoluenesulfonic acid. As inorganic acids, hydrochloric acid, sulfuric acid and nitric acid may be exemplified.

Appropriate solvents to be used in the deprotection reaction may include, for instance, water and dioxane, but the present invention may not be restricted by this particularly.

The acid addition salt of δ-aminolevulinic acid (I) thus prepared is neutralized by an alkali such as sodium hydroxide, as disclosed in Unexamined Published Japanese Patent Application No. 2-76841, thereby obtaining δ-aminolevulinic acid.

### [Example]

The present invention will be explained in detail referring to example.

### Example 1

### (A) Preparation of N-tetrahydrofurfuryl phthalimide

19.8g (134 mmol) of phthalic anhydride were dissolved in 500 ml of chloroform. To the resultant solution, 10g (99 mmol) of tetrahydrofurfurylamine were added with stirring. The resultant mixture was subjected to a reflux overnight while the reactant water yielded was distilled off. Thus obtained reaction mixture was allowed to cool and then was poured into 300 ml of aqueous solution of saturated sodium hydrogencarbonate. Thereafter, an organic solvent layer was separated from the reaction mixture. The residual water layer was subjected to an extraction twice with chloroform and the extract thus obtained was combined with the organic solvent layer which was previously separated.

Thereafter thus combined organic solvent layer was washed with aqueous solution of sodium hydrogencarbonate and water in order, followed by drying with anhydrous magnesium sulfate. From thus dried organic solvent layer, the solvent was distilled off under a reduced pressure to obtain crude product. Then the crude product was purified by recrystallization from a mixture solvent of hexane and methylene chloride, thereby forming N-tetrahydrofurfuryl phthalimide. Yield of the product and the properties thereof are as follows:
N-tetrahydrofurfuryl phthalimide
Yield: 21.8g (yield 95.2%)
Melting point: 86.5 - 87.5°C
¹H-NMR(CDCℓ₃) : δ
7.9 - 7.8 (2H, m, aromatic-H),
7.75 - 7.65 (2H, m, aromatic-H),
4.3 - 4.2 (1H, m),
4.0 - 3.6 (4H, m),
2.1 - 1.8 (3H, m),
1.75 - 1.6 (1H, m)

### (B) Preparation of 4-phthalimidomethyl-4-butanolide and 5-phthalimidolevulinic acid

To a biphasic solution consisting of 25 ml of carbon tetrachloride, 25 ml of acetonitrile and 30 ml of water, which was dissolving 5.0g (220 mmol) of N-tetrahydrofurfuryl phthalimide prepared in the above step (A), 19g (87 mmol) of sodium periodate in the form of powder and 0.10g (2.2 mol%) of ruthenium chloride hydrate were added, followed by stirring vigorously overnight at an ambient temperature. Upon completion of the reaction, insoluble matter was filtered off. Then the filtrate was subjected to a vacuum distillation to remove the solvent. Thus yielded residue was dissolved by a mixture solution consisting of chloroform and 1N hydrochloric acid aqueous solution, followed by an extraction with chloroform. The organic solvent layer of the extract was dried using anhydrous magnesium sulfate. From thus dried organic solvent layer, the solvent was distilled off under a reduced pressure thereby, obtaining a residue. The residue was purified by column chromatograpahy on silica gel using a solvent mixture (chloroform:methanol = 95:5 v/v) as an eluent, thereby obtaining 4-phthalimidomethyl-4-butanolide. Subsequently using the same column, column chromatography was performed by using an other solvent mixture (chloroform:methanol:formic acid = 95:4:1 v/v) as an eluent, thereby obtaining 5-phthalimidolevulinic acid. Yields of thus obtained products and properties thereof are as follows:
4-phthalimidomethyl-4-butanolide
Yield: 1.5g (yield 28%)
Melting point: 170 - 171°C
¹H - NMR (CDCℓ₃) : δ
7.91 - 7.83 (2H, m, aromatic-H),
7.78 - 7.72 (2H, m, aromatic-H),
4.87 (1H, dq, J = 5.3, 7.1 Hz, H-4)
4.02 (1H, dd, J = 14.2, 7.7 Hz, H-5),
3.84 (1H, dd, J = 14.2, 5.2 Hz, H-5),
2.71 - 2.49 (2H, m, H-2),
2.46 - 2.34 (1H, m, H-3),
2.13 - 2.03 (1H, m, H-3)
5-phthalimidolevulinic acid
Yield: 2.1g (yield 37%)
Melting point: 160 - 162°C
¹H - NMR (CDCℓ₃ - DMSO - d₆) : δ
7.88 - 7.83 (2H, m, aromatic-H),
7.78 - 7.73 (2H, m, aromatic-H),
4.57 (2H, s, H-5),
2.85 (2H, t, J = 6.6 Hz, H-3),
2.64 (2H, t, J = 6.6 Hz, H-2)

### (C) Preparation of 5-phthalimidolevulinic acid

To a biphasic solution consisting of 2.0 ml of carbon tetrachloride, 10 ml of acetonitrile and 3.0 ml of water, which was dissolving 0.30g (1.2 mmol) of 4-phthalimidomethyl-4-butanolide prepared in the above step (B), 2.5g (12 mmol) of sodium periodate in the form of powder and 90 mg (30 mol%) of ruthenium chloride hydrate were added, followed by stirring vigorously at 50°C for 24 hours. Upon completion of the reaction, the reaction mixture was subjected to a vacuum distillation to remove the solvent.

Then the residue thus obtained was dissolved by a mixture solution consisting of chloroform and 1N hydrochloric acid aqueous solution, followed by an extraction with chloroform. The organic solvent layer of the extract was dried by anhydrous magnesium sulfate. From thus dried organic solvent layer, the solvent was distilled off under a reduced pressure, thereby obtaining a residue. The residue was purified by column chromatography on silica gel using a solvent mixture (chloroform:methanol:formic acid = 95:4:1 v/v) as an eluent, thereby obtaining 5-phthalimidolevulinic acid. Yield of thus obtained compound and properties thereof are as follows:
5-phthalimidolevulinic acid
Yield: 32 mg (yield 10%)
Melting point: 160 - 162°C
¹H - NMR (CDCℓ₃ - DMSO - d₆) : δ
7.88 - 7.83 (2H, m, aromatic-H),
7.78 - 7.73 (2H, m, aromatic-H),
4.57 (2H, s, H-5),
2.85 (2H, t, J = 6.6 Hz, H-3),
2.64 (2H, t, J = 6.6 Hz, H-2)

### (D) Preparation of 5-aminolevulinic acid hydrochloride

2.1g (8.0 mmol) of 5-phthalimidolevulinic acid prepared in the previous step (B) or (C) were suspended in 100 ml of 6N hydrochloric acid aqueous solution and the resultant was subjected to a reflux for 8 hours. Upon completion of the reaction, the reaction solution was cooled to an ambient temperature. After the deposited crystals were filtered off, the filtrate was subjected to a vacuum distillation to remove the solvent, thereby obtaining a residue. The residue was purified by the recrystallization from ethanol-water, thereby forming 5-aminolevulinic acid hydrochloride. Yield of the compound thus prepared and properties thereof are as follows:
5-aminolevulinic acid hydrochloride
Yield: 0.861g (yield 63.8%)
Melting point: 142 - 145°C (149 - 151°C in the literature)
¹H - NMR (D₂O) : δ
4.07 (2H, s, H-5),
2.84 (2H, t, J = 6.3 Hz, H-3),
2.66 (2H, t, J = 6.2 Hz, H-2)

### Example 2

To a biphasic solution consisting of 2 ml of carbon tetrachloride, 10 ml of acetonitrile and 3 ml of water, which is dissolving 0.30g (1.3 mmol) of N-tetrahydrofurfuryl phthalimide prepared in the previous step (A) of Example 1, 1.7g (7.8 mmol) of sodium periodate in the form of powder and 8.0 mg (2.2 mol%) of ruthenium chloride hydrate. The resultant was stirred vigorously overnight at 80°C. Upon completion of the reaction, the reaction mixture was subjected to a vacuum distillation to remove the solvent. Thus obtained residue was dissolved by a mixture solution consisting of chloroform and 1N hydrochloric acid aqueous solution, followed by an extraction with chloroform. The organic solvent layer of the extract was dried using anhydrous magnesium sulfate. From thus dried organic solvent layer, the solvent was distilled off under a reduced pressure, thereby obtaining a residue. The residue was purified by column chromatography on silica gel using a solvent mixture (chloroform:methanol:formic acid = 95:4:1 v/v) as an eluent, thereby obtaining 5-phthalimidolevulinic acid. As a result, 5-phatalimidolevulinic acid can be prepared without via 4-phthalimidomethyl-4-butanolide as seen in Example 1. Yield of the compound thus prepared and properties thereof are as follows:
5-phthalimidolevulinic acid
Yield: 0.20g (yield 59%)
Melting point: 160 - 162°C
¹H - NMR (CDCℓ₃ - DMSO - d₆) : δ
7.88 - 7.83 (2H, m, aromatic-H),
7.78 - 7.73 (2H, m, aromatic-H),
4.57 (2H, s, H-5),
2.85 (2H, t, J = 6.6 Hz, H-3),
2.64 (2H, t, J = 6.6 Hz, H-2)
The amino-protecting group thereof was deprotected as according to the process similar to one as describe in the step (D) of Example 1 to yield 5-aminolevulinic acid hydrochloride, which showed the similar properties as that of Example 1.

From the above results, it was confirmed that 5-aminolevulinic acid hydrochloride can be prepared without forming the reaction intermediate, i.e., 4-phthalimidomethyl-4-butanolide , by the oxidation using ruthenium chloride hydrate at the reaction temperature which is higher than that of Example 1, i.e., 80°C or more.

### Example 3

### (A) Preparation of N-benzoyltetrahydrofurfurylamide

To a benzene solution which was dissolving 10 ml (97 mmol) of tetrahydrofurfurylamine and 15 ml (107 mmol) of triethylamine, 11 ml (97 mmol) of benzoyl chloride was added dropwise slowly at 0°C under anhydrous conditions. Upon completion of adding, the resultant solution was stirred overnight at an ambient temperature. When the reaction terminated the reaction mixture was subjected to a vacuum distillation to remove the solvent, thereby obtaining a residue. Thereafter the residue was purified by recrystallization from a mixture solvent consisting of n-hexane and ethylacetate, thereby forming N-benzoyltetrahydrofurfurylamide. Yield of thus prepared compound and properties thereof are as follows:
N-benzoyltetrahydrofurfurylamide
Yield: 16g (yield 77%)
Melting point: 93 - 94°C
¹H - NMR (CDCℓ₃) : δ
7.80 - 7.86 (2H, m, aromatic-H),
7.52 - 7.38 (2H, m, aromatic-H),
6.61 (1H, br, NH),
4.07 (1H, dq, J = 7.1, 3.3 Hz, H-4),
3.93 - 3.72 (3H, m, H-1, H-5),
3.35 (1H, ddd, J = 13.4, 7.7, 5.3 Hz, H-5),
2.09 - 1.86 (3H, m, H-2, H-3),
1.68 - 1.54 (1H, m, H-3)

### (B) Preparation of 5-benzoylamidopentane-4-olide and 5-benzoylamidolevulinic acid

To a biphasic solution consisting of 2 ml of carbon tetrachloride, 10 ml of acetonitrile and 3 ml of water, which was dissolving 0.3g (1.46 mmol) of N-bensoyltetrahydrofurfurylamide prepared in the above step (A), 1.9g (8.8 mmol) of sodium periodate in the form of powder and 8 mg (2.2 mol%) of ruthenium chloride hydrate. The resultant mixture was stirred vigorously overnight at an ambient temperature. Upon completion of the reaction, insoluble matter in the reaction solution was filtered off. Then the filtrate was subjected to a vacuum distillation to remove the solvent. Thus obtained residue was dissolved by a mixture solution consisting of chloroform and 1N hydrochloric acid aqueous solution, followed by an extraction using chloroform. Subsequently the organic solvent layer of the extract was dried with anhydrous magnesium sulfate. From thus dried organic solvent layer, chloroform was distilled off under an reduced pressure, thereby obtaining a residue. The residue was purified by column chromatography on silica gel using a solvent mixture (chloroform:methanol = 30:1 v/v) as an eluent, thereby obtaining 5-benzoylamidopentane-4-olide. Subsequently using the same column, column chromatography was performed by using an other eluate (chloroform:methanol:formic acid = 18:1:1 v/v), thereby obtaining 5-benzoylamidolevulinic acid. Yields of thus prepared compounds and properties thereof are as follows:
5-benzoylamidopentan-4-olide
Yield: 0.076g (yield 24.0%)
Melting point: 129 - 130°C
¹H - NMR (CDCℓ₃) : δ
7.83 - 7.77 (2H, m, aromatic-H),
7.54 - 7.37 (3H, m, aromatic-H),
7.02 (1H, br, NH),
4.73 (1H, dq, J = 7.3, 3.4 Hz, H-4),
3.90 (1H, ddd, J = 14.5, 6.6, 3.2 Hz, H-5),
3.54 (1H, ddd, J = 14.5, 7.0, 5.6 Hz, H-5),
2.59 - 2.51 (2H, m, H-2),
2.41 - 2.28 (1H, m, H-3),
2.09 - 1.94 (1H, m, H-3)
5-benzolamidolevulinic acid
Yield: 0.075g (yield 21.8%)
Melting point: 120 - 122°C
¹H - NMR (CD₃OD) : δ
7.85 (2H, d, J = 7.1 Hz, aromatic-H),
7.55 (1H, t, J = 7.2 Hz, aromatic-H),
7.46 (2H, t, J = 7.3 Hz, aromatic-H),
4.26 (2H, s, H-5),
2.80 (3H, t, J = 6.3 Hz, H-3)
2.61 (2H, t, J = 6.4 Hz, H-2)
5-benzolamidopentane-4-olide obtained here was further oxidized in the same way as in the step (C) of Example 1, to give 5-benzoylamidolevulinic acid.

### (C) Preparation of 5-aminolevulinic acid hydrochloride

0.874g (3.72 mmol) of 5-benzolamidolevulinic acid prepared in the step (B) was suspended in 10 ml of 6N hydyrochloric acid aqueous solution and was subjected to a reflux for 7 hours. Upon completion of the reaction, the reaction mixture was cooled until an ambient temperature and the deposited crystals were filtered off. Then from the filtrate, the solvent was distilled off under a reduced pressure. Thereafter the residue thus obtained was purified by recrystallization from hydrous ethanol of 10 wt%, thereby forming 5-aminolevulinic acid hydrochloride. Yield of thus obtained compound and properties thereof are as follows:
5-aminolevulinic acid hydrochloride
Yield: 0.390g (yield 62.5%)
¹H - NMR (D₂O) : δ
4.07 (2H, s, H-5),
2.84 (2H, t, J = 6.3 Hz, H-3),
2.66 (2H, t, J = 6.2 Hz, H-2)

## Claims

1. A method of preparing an acid addition salt (I) of δ-aminolevulinic acid of the following formula 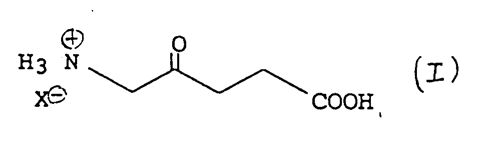 wherein X stands for a monovalent organic or inorganic acid radical, comprising:
(a) introducing an amino-protecting group into an amino group of tetrahydrofurfurylamine (IV) of the following formula, thereby obtaining a compound (III) of the following formula: 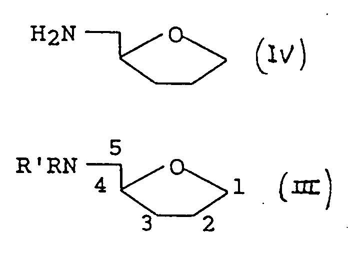 wherein, R and R' respectively represent a common amino-protecting group or a hydrogen atom and at least one of R and R' is the amino-protecting group; when both R and R' are the amino-protecting groups as defined above, R and R' may be linked each other to form a ring;
(b) oxidizing carbon atoms of the first- and fourth-positions of Said compound (III) which is prepared in the above step (a), thereby obtaining a compound (II) of the following formula: 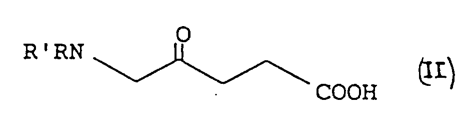 wherein R and R' are same means as described above; and
(c) deprotecting said amino-protecting group of said compound (II) which is prepared in said step (b), by an acid, thereby obtaining the compound (I).

2. A method according to claim 1, characterized in that said amino-protecting group to be introduced into said amino group of tetrahydrofurfurylamine (IV) in said step (a) is an acyl group or a siliyl group.

3. A method according to claim 1, characterized in that said oxidation of the first- and fourth-position carbon atoms of said compound (III) in said step (b) is conducted in the presence of ruthenium oxide or ruthenium trichloride with a strong oxidizing agent together.

4. A method according to claim 3, characterized in that said ruthenium oxide is one selected from the group consisting of ruthenium tetraoxide and ruthenium dioxide.

5. A method according to claim 3, characterized in that said strong oxidizing agent is one selected from the group consisting of sodium periodate, potassium periodate, sodium hypochlorite and sodium bromate.

6. A method according to claim 3, characterized in that said oxidation in said step (b) is performed at a reaction temperature of not lower than 80°C.

7. A method according to claim 1, characterized in that said oxidation of the first- and fourth-position carbon atoms of said compound (III) in said step (b) is conducted by a chromic acid series oxidizing agent.

8. A method according to claim 7, characterized in that said chromic acid series oxidizing agent is one selected from the group consisting of chromium trioxide and t-butyl chromate.

9. A method according to claim 1, characterized in that said acid to be used for deprotecting said amino-protecting group of said compound (II) in said step (c) is an organic acid.

10. A method according to claim 9, characterized in that said organic acid is one selected from the group consisting of acetic acid, trifluoroacetic acid and paratoluenesulfonic acid.

11. A method according to claim 1, characterized in that said acid to be used for deprotecting said amino-protecting group of said compound (II) in said step (c) is an inorganic acid.

12. A method according to claim 11, characterized in that said inorganic acid is one selected from the group consisting of hydrochloric acid, sulfuric acid and nitric acid.

## Patentansprüche

1. Verfahren zur Herstellung eines Säureadditionssalzes (I) der δ-Aminolävulinsäure der nachfolgenden Formel 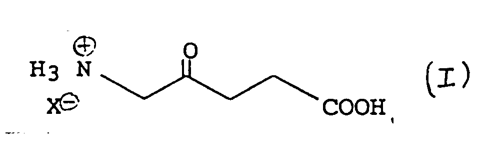 wobei X ein einwertiger organischer oder anorganischer Säurerest ist, umfassend:
(a) Einführen einer Aminoschutzgruppe in eine Aminogruppe von Tetrahydrofurfurylamin (IV) der nachfolgenden Formel, wodurch eine Verbindung (III) der nachfolgenden Formel erhalten wird: 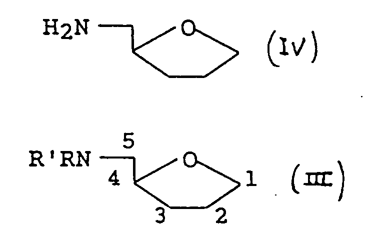 wobei R bzw. R' eine übliche Aminoschutzgruppe oder ein Wasserstoffatom bedeutet und wenigstens einer der Reste R und R' die Aminoschutzgruppe darstellt und, wenn sowohl R als auch R' die oben definierten Aminoschutzgruppen sind, R und R' miteinander zur Bildung eines Ringes verbunden sein können;
(b) Oxidieren der Kohlenstoffatome an den Positionen 1 und 4 der Verbindung (III), die im oben genannten Schritt (a) hergestellt wurde, wodurch eine Verbindung (II) der nachfolgenden Formel erhalten wird: 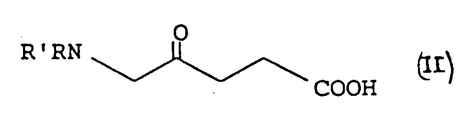 wobei R und R' die gleichen, oben beschriebenen Bedeutungen aufweisen; und
(c) Entfernen der Aminoschutzgruppe von der Verbindung (II), die im Schritt (b) hergestellt wurde, durch eine Säure, wodurch die Verbindung (I) erhalten wird.

2. Verfahren nach Anspruch 1, dadurch gekennzeichnet, daß die in die Aminogruppe von Tetrahydrofurfurylamin (IV) im Schritt (a) einzuführende Aminoschutzgruppe eine Acylgruppe oder eine Siliylgruppe ist.

3. Verfahren nach Anspruch 1, dadurch gekennzeichnet, daß die Oxidation der Kohlenstoffatome der Verbindung (III) in der Position 1 und 4 im Schritt (b) in Gegenwart eines Rutheniumoxids oder von Rutheniumtrichlorid zusammen mit einem starken Oxidationsmittel durchgeführt wird.

4. Verfahren nach Anspruch 3, dadurch gekennzeichnet, daß das Rutheniumoxid ausgewählt wird aus der Gruppe, bestehend aus Rutheniumtetraoxid und Rutheniumdioxid.

5. Verfahren nach Anspruch 3, dadurch gekennzeichnet, daß das starke Oxidationsmittel aus der Gruppe ausgewählt wird, bestehend aus Natriumperiodat, Kaliumperiodat, Natriumhypochlorit und Natriumbromat.

6. Verfahren nach Anspruch 3, dadurch gekennzeichnet, daß die Oxidation im Schritt (b) bei einer Reaktionstemperatur von nicht unter 80°C durchgeführt wird.

7. Verfahren nach Anspruch 1, dadurch gekennzeichnet, daß die Oxidation der Kohlenstoffatome der Positionen 1 und 4 der Verbindung (III) im Schritt (b) durch ein Oxidationsmittel der Chromsäurereihe durchgeführt wird.

8. Verfahren nach Anspruch 7, dadurch gekennzeichnet, daß das Oxidationsmittel der Chromsäurereihe ausgewählt wird aus der Gruppe, bestehend aus Chromtrioxid und t-Butylchromat.

9. Verfahren nach Anspruch 1, dadurch gekennzeichnet, daß die zur Entfernung der Aminoschutzgruppe der Verbindung (II) im Schritt (c) verwendete Säure eine organische Säure ist.

10. Verfahren nach Anspruch 9, dadurch gekennzeichnet, daß die organische Säure ausgewählt wird aus der Gruppe, bestehend aus Essigsäure, Trifluoressigsäure und Paratoluolsulfonsäure.

11. Verfahren nach Anspruch 1, dadurch gekennzeichnet, daß die zur Entfernung der Aminoschutzgruppe der Verbindung (II) im Schritt (c) verwendete Säure eine anorganische Säure ist.

12. Verfahren nach Anspruch 11, dadurch gekennzeichnet, daß die anorganische Säure ausgewählt wird aus der Gruppe, bestehend aus Salzsäure, Schwefelsäure und Salpetersäure.

## Revendications

1. Procédé de fabrication d'un sel d'addition d'acide de l'acide δ-aminolévulinique de formule (I) suivante : 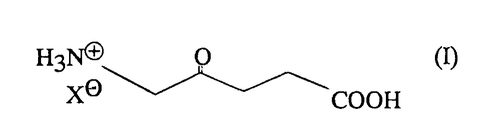 dans laquelle X représente un radical monovalent d'acide organique ou inorganique, comprenant :
(a) l'introduction d'un groupe amino-protecteur sur le groupe amino de la tétrahydrofurfurylamine de formule (IV) suivante, pour obtenir ainsi un composé de formule (III) suivante : 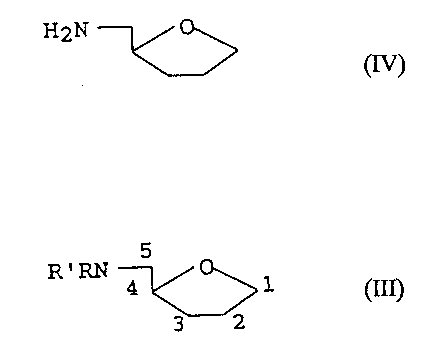 dans lesquelles R et R' représentent respectivement un groupe amino-protecteur courant ou un atome d'hydrogène et l'un au moins des restes R et R' est le groupe amino-protecteur; lorsque R et R' sont tous deux les groupes amino-protecteurs définis ci-dessus, R et R' peuvent être reliés l'un à l'autre pour former un cycle;
(b) l'oxydation des atomes de carbone en positions 1 et 4 dudit composé (III) préparé dans l'étape (a) ci-dessus, pour obtenir ainsi un composé de formule (II) suivante : 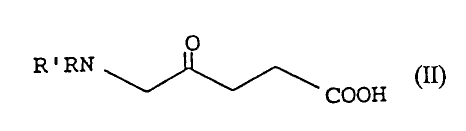 dans laquelle R et R' sont définis comme ci-dessus et
(c) l'élimination dudit groupe amino-protecteur dudit composé (II) préparé dans ladite étape (b), par un acide, pour obtenir ainsi le composé (I).

2. Procédé selon la revendication 1, caractérisé en ce que ledit groupe amino-protecteur à introduire sur ledit groupe amino de la tétrahydrofurfurylamine (IV) dans ladite étape (a) est un groupe acyle ou un groupe silyle.

3. Procédé selon la revendication 1, caractérisé en ce que ladite oxydation des atomes de carbone en positions 1 et 4 dudit composé (III) dans ladite étape (b) est effectuée par un agent oxydant fort en présence d'oxyde de ruthénium ou le trichlorure de ruthénium.

4. Procédé selon la revendication 3, caractérisé en ce que ledit oxyde de ruthénium est choisi parmi le tétroxyde de ruthénium et le dioxyde de ruthénium.

5. Procédé selon la revendication 3, caractérisé en ce que ledit agent oxydant fort est choisi parmi le periodate de sodium, le periodate de potassium, l'hypochlorite de sodium et le bromate de sodium.

6. Procédé selon la revendication 3, caractérisé en ce que ladite oxydation dans ladite étape (b) est effectuée à une température de réaction de pas moins de 80°C.

7. Procédé selon la revendication 1, caractérisé en ce que ladite oxydation des atomes de carbone en position 1 et 4 dudit composé (III) dans ladite étape (b) est effectuée par un agent oxydant de la série de l'acide chromique.

8. Procédé selon la revendication 7, caractérisé en ce que ledit agent oxydant de la série de l'acide chromique est choisi parmi le trioxyde de chrome et le chromate de t-butyle.

9. Procédé selon la revendication 1, caractérisé en ce que ledit acide à utiliser pour l'élimination dudit groupe amino-protecteur dudit composé (II) dans ladite étape (c) est un acide organique.

10. Procédé selon la revendication 9, caractérisé en ce que ledit acide organique est choisi parmi l'acide acétique, l'acide trifluoroacétique et l'acide paratoluènesulfonique.

11. Procédé selon la revendication 1, caractérisé en ce que ledit acide à utiliser pour l'élimination dudit groupe amino-protecteur dudit composé (II) dans ladite étape (c) est un acide inorganique.

12. Procédé selon la revendication 11, caractérisé en ce que ledit acide inorganique est choisi parmi l'acide chlorhydrique, l'acide sulfurique et l'acide nitrique.
